(19) Europäisches Patentamt
European Patent Office
Office européen des brevets

(11) Veröffentlichungsnummer: **0 022 059**
**B1**

# (12) EUROPÄISCHE PATENTSCHRIFT

(45) Veröffentlichungstag der Patentschrift: **17.08.83**

(21) Anmeldenummer: **80810146.3**

(22) Anmeldetag: **01.05.80**

(51) Int. Cl.³: **C 07 C 101/22,** C 07 D 295/12, C 07 D 233/61, C 07 C 99/00, C 08 G 59/52

## (54) N-substituierte Asparaginsäureester, ihre Herstellung und Verwendung.

(30) Priorität: **08.05.79 CH 4306/79**

(43) Veröffentlichungstag der Anmeldung: **07.01.81 Patentblatt 81/1**

(45) Bekanntmachung des Hinweises auf die Patenterteilung: **17.08.83 Patentblatt 83/33**

(84) Benannte Vertragsstaaten: **CH DE FR GB IT LI NL**

(56) Entgegenhaltungen:
**DE-A-2 341 045**
**DE-B-1 907 149**
**GB-A-960 622**

(73) Patentinhaber: **CIBA-GEIGY AG, Patentabteilung Postfach, CH-4002 Basel (CH)**

(72) Erfinder: **Stockinger, Friedrich, Stutzrain 4, CH-4434 Hölstein (CH)**
Erfinder: **Eldin, Sameer H., Dr., Am Strausee 27/18, CH-4127 Birsfelden (CH)**
Erfinder: **Lohse, Friedrich, Prof. Dr., Buchenstrasse 23, CH-4104 Oberwil (CH)**

**EP 0 022 059 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

N-substituierte Asparaginsäureester, ihre Herstellung und Verwendung

Gegenstand der vorliegenden Erfindung sind N-substituierte Asparaginsäuremonoester, Verfahren zu ihrer Herstellung und ihre Verwendung als Beschleuniger bei der Härtung von Epoxidharzen oder als latente Härtungsmittel für Epoxidharze.

Es ist bekannt, bei der Härtung von Epoxidharzen mit Polycarbonsäureanhydriden oder Dicyandiamid den härtbaren Gemischen Härtungsbeschleuniger, wie z. B. tertiäre Amine, zuzusetzen. Wie aus der GB-PS 1 050 678 hervorgeht, bewirken Zusätze von tertiären Aminen, wie z. B. Benzyldimethylamin, bei der Härtung von Epoxidharzen mit Polycarbonsäureanhydriden eine Verkürzung der Härtungszeit, doch keine Erniedrigung der relativ hohen Härtungstemperaturen. Außerdem verschlechtern sich die Eigenschaften der gehärteten Epoxidharze, wenn man tertiäre Amine als Beschleuniger zusetzt. Um diesen Nachteilen zu begegnen, wird in der GB-PS 1 050 678 die Verwendung von Imidazolen als Härtungsbeschleuniger für die Anhydridhärtung von Epoxidharzen vorgeschlagen. Imidazole stellen im Vergleich zu anderen bekannten Beschleunigern sehr wirksame Beschleuniger dar, jedoch weisen Imidazole enthaltende Einkomponentensysteme aus Epoxidharzen und Polycarbonsäureanhydriden, wie Preßmassen oder Sinterpulver, den Nachteil einer ungenügenden Lagerstabilität auf.

Desgleichen ist die aus »Journal of Polymer Science«, Teil A-1, Bd. 5, S. 1609–1617 (1967), bekannte Härtung von Epoxidharzen mit Dicyandiamid in Gegenwart von tertiären Aminen als Härtungsbeschleuniger mit Nachteilen verbunden. Die üblichen beschleunigenden Zusätze von tertiären Aminen, welche bei der Härtung von Epoxidharzen mit Polycarbonsäureanhydriden eine starke Beschleunigung der Gelierung und Härtung zur Folge haben, bringen bei der Verwendung von Dicyanadiamid als Härtungsmittel nur eine schwache Beschleunigung. Durch Zusätze von größeren Mengen Beschleuniger kann die Härtungszeit wohl verkürzt werden, die Lagerstabilität der Epoxidharz-Dicyandiamid-Gemische geht aber weitgehend oder vollständig verloren. In den meisten Fällen wird Dicyandiamid aber gerade wegen der guten Lagerstabilität der härtbaren Epoxidharzmischungen als Härtungsmittel für Epoxidharze eingesetzt.

Es wurde nun gefunden, daß N-substituierte Asparaginsäuremonoester wertvolle Härtungsbeschleuniger für die Härtung von Epoxidharzen mit sowohl Polycarbonsäureanhydriden als auch mit Dicyandiamid darstellen, da sie in Einkomponentensystemen oben geschilderten Nachteil bezüglich ungünstiger Lagerstabilität nicht aufweisen.

Gegenstand der vorliegenden Erfindung sind somit neue N-substituierte Asparaginsäuremonoester der Formel I

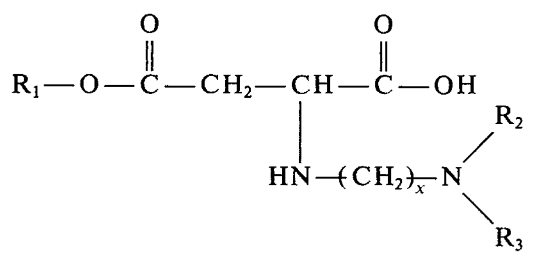

(I)

worin

| | |
|---|---|
| $R_1$ | ein gegebenenfalls Äthersauerstoffatome enthaltendes Alkyl mit 1 bis 12 C-Atomen, Cyclohexyl, Phenyl, Tolyl oder Benzyl bedeutet, |
| x | für die Zahl 2 oder 3 steht, |
| $R_2$ und $R_3$ | unabhängig voneinander je ein Methyl oder Äthyl oder zusammen mit dem N-Atom einen N-heterocyclischen Ring der Formel |

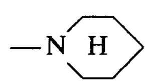

oder

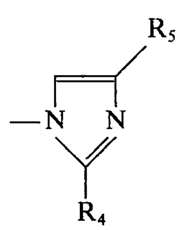

bedeuten, worin $R_4$ und $R_5$ unabhängig voneinander je ein Wasserstoffatom, Methyl oder Äthyl bedeuten.

Vorzugsweise bedeuten in der Formel I $R_1$ ein Alkyl mit 1 bis 6 C-Atomen oder Cyclohexyl, x die Zahl 3 und $R_2$ und $R_3$ je ein Methyl oder Äthyl, insbesondere je ein Methyl, oder zusammen mit dem N-Atom

einen N-heterocyclischen Ring der Formel

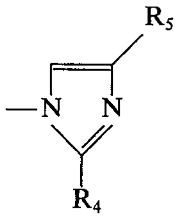

worin $R_4$ für Methyl steht und $R_5$ ein Wasserstoffatom oder Äthyl, insbesondere ein Wasserstoffatom bedeutet.

Als Alkyl kann $R_1$ beispielsweise die folgende Bedeutung haben:

$CH_3-(CH_2)_y-$ $y = 0$ bis 11, 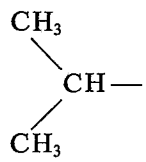 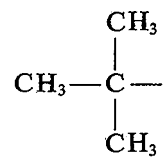

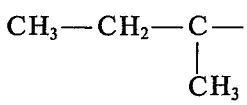 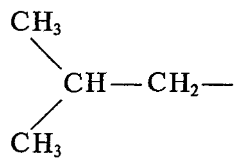 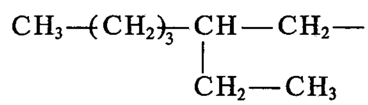

und

$CH_3-CH_2-(O-CH_2-CH_2)_z-$ $z = 1$ bis 3.

Die N-substituierten Asparaginsäuremonoester der Formel I können gemäß den in »Journal of Organic Chemistry«, Bd. 24 (1959), S. 1096–98, und in »Canadian Journal of Chemistry«, Bd. 40 (1962), S. 163–5, beschriebenen Verfahren hergestellt werden, indem man an 1 Mol eines Maleinsäuremonoesters der Formel II

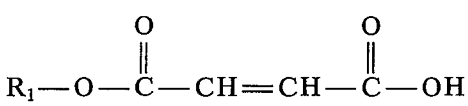
(II)

worin $R_1$ die gleiche Bedeutung wie in Formel I hat, 1 Mol eines Amins der Formel III

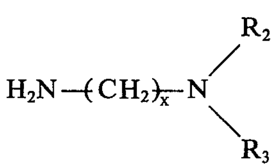
(III)

worin $R_2$, $R_3$ und x die gleiche Bedeutung wie in Formel I haben, in Gegenwart eines tertiären Amins anlagert.

Vorzugsweise geht man bei der Herstellung der erfindungsgemäßen Verbindungen der Formel I von Maleinsäuremonoestern der Formel II aus, worin $R_1$ ein Alkyl mit 1 bis 6 C-Atomen oder Cyclohexyl bedeutet, und setzt diese vorzugsweise mit den Aminen der Formel III, worin $R_2$ und $R_3$ je ein Methyl oder Äthyl oder zusammen mit dem N-Atom einen N-heterocyclischen Ring der Formel

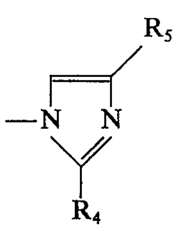

bedeuten, worin $R_4$ für Methyl steht und $R_5$ ein Wasserstoffatom oder Äthyl bedeutet und x für 3 steht, in Gegenwart von Triäthylamin oder Pyridin, insbesondere Triäthylamin, um.

Die Maleinsäuremonoester der Formel II sind bekannt (siehe z. B. »Canadian Journal of Chemistry, Bd. 40 [1962], S. 163–5). Desgleichen stellen die Amine der Formel III bekannte Verbindungen dar.

Wie eingangs erwähnt, stellen die Asparaginsäurederivate wertvolle Härtungsbeschleuniger für die Härtung von Epoxidharzen mit Polycarbonsäureanhydriden oder mit Dicyandiamid dar. Die erfindungsgemäßen Asparaginsäurederivate eignen sich auch als katalytische Härtungsmittel für Epoxidharze. Die die erfindungsgemäßen Asparaginsäurederivate enthaltenden härtbaren Epoxidharzmischungen zeichnen sich durch hervorragende Lagerstabilitäten bei Raumtemperatur aus und weisen, verglichen mit bekannten Härtungsbeschleuniger enthaltenden Epoxidharzmischungen, stark verbesserte Latenzfaktoren auf. Unter Latenzfaktor versteht man den Quotienten aus Gelierzeit bei niedriger Temperatur und der Gelierzeit bei höherer Temperatur

$$\left(\text{Latenzfaktor} = \frac{\text{Gelierzeit bei niedriger Temperatur}}{\text{Gelierzeit bei höherer Temperatur}}\right)$$

wobei man in der Wahl der höheren Temperatur frei ist, sich aber generell an entsprechende Verarbeitungstemperaturen der härtbaren Epoxidharzmischung hält.

Gegenstand der vorliegenden Erfindung ist daher auch die Verwendung der Asparaginsäuremonoester der Formel I als Härtungsbeschleuniger oder katalytisch wirkendes Härtungsmittel für Epoxidharze.

Bei der Härtung von Epoxidharzen mit Polycarbonsäureanhydriden verwendet man die erfindungsgemäßen Härtungsbeschleuniger in Mengen von 0,5 bis 2 Gewichts-%, vorzugsweise 1 bis 1,5 Gewichts-%, bezogen auf das Epoxidharzgewicht.

Als Härtungsbeschleuniger bei der Härtung von Epoxidharzen mit Dicyandiamid werden die erfindungsgemäßen Asparaginsäurederivate in Mengen von 0,1 bis 1,0 Gewichts-%, vorzugsweise von 0,3 bis 0,5 Gewichts-%, bezogen auf den Anteil Epoxidharz, eingesetzt.

Für die katalytische Härtung von Epoxidharzen verwendet man die erfindungsgemäßen Asparaginsäurederivate in Mengen von 2 bis 10 Gewichts-%, vorzugsweise von 3,5 bis 6 Gewichts-%, bezogen auf den Anteil Epoxidharz.

Als Epoxidharze, zu deren Härtung die erfindungsgemäßen Asparaginsäurederivate als Härtungsbeschleuniger eingesetzt werden können, eignen sich alle bekannten Klassen von Epoxidharzen. Vor allem kommen Epoxidverbindungen mit durchschnittlich mehr als einer an ein Heteroatom (z. B. Schwefel, vorzugsweise Sauerstoff oder Stickstoff) gebundenen Glycidylgruppe, $\beta$-Methylglycidylgruppe oder 2,3-Epoxycyclopentylgruppe in Frage; genannt seien insbesondere Bis-(2,3-epoxycyclopentyl)-äther; Di- bzw. Polyglycidyläther von mehrwertigen aliphatischen Alkoholen, wie 1,4-Butandiol, oder Polyalkylenglykolen, wie Polypropylenglykole; Di- oder Polyglycidyläther von cycloaliphatischen Polyolen, wie 2,2-Bis-(4-hydroxycyclohexyl)-propan; Di- bzw. Polyglycidyläther von mehrwertigen Phenolen, wie Resorcin, Bis-(p-hydroxyphenyl)methan, 2,2-Bis(p-hydroxyphenyl)-propan (=Diomethan), 2,2-Bis-(4'-hydroxy-3',5'-dibromphenyl)propan, 1,1,2,2-Tetrakis-(p-hydroxylphenyl)-äthan, oder von unter sauren Bedingungen erhaltenen Kondensationsprodukten von Phenolen mit Formaldehyd, wie Phenol-Novolake und Kresol-Novolake; Di- bzw. Poly-($\beta$-methylglycidyl)äther der oben angeführten mehrwertigen Alkohole oder mehrwertigen Phenole; Polyglycidylester von mehrwertigen Carbonsäuren, wie Phthalsäure, Terephthalsäure, $\Delta^4$-Tetrahydrophthalsäure und Hexahydrophthalsäure; N-Glycidylderivate von Aminen, Amiden und heterocyclischen Stickstoffbasen, wie

N,N-Diglycidylanilin, N,N-Diglycidyltoluidin,
N,N,N',N'-Tetraglycidyl-bis(p-amino-phenyl)-methan; Triglycidylisocyanurat;
N,N'-Diglycidyläthylenharnstoff; N,N'-Diglycidyl-5,5-dimethylhydantoin,
N,N'-Diglydidyl-5-iso-propylhydatoin;
N,N'-Diglycidyl-5,5-dimethyl-6-isopropyl-5,6-dihydro-uracil.

Geeignete Epoxidverbindungen sind ferner alicyclische Diepoxide, wie Limonendioxyd, Dicyclopentadiendioxyd, Äthylenglykol-bis-(3,4-epoxytetrahydrodocyclopentadien-8-yl)-glycidyläther, sowie Verbindungen mit zwei Epoxycyclohexylresten, wie

Diäthylenglykol-bis-(3,4-epoxycyclohexancarboxylat),
Bis-3,4-(epoxycyclohexylmethyl)-succinat,
3',4'-Epoxy-6'-methylcyclohexylmethyl-3,4-epoxy-6-methyl-cyclohexan-carboxylat und
3',4'-Epoxyhexahydrobenzal-3,4-epoxycyclo-hexan-1,1-dimethanol.

Als geeignete Polycarbonsäureanhydride, die zusammen mit den erfindungsgemäßen Härtungsbeschleunigern zum Härten von Epoxidharzen eingesetzt werden können, seien beispielsweise genannt: cycloaliphatische Polycarbonsäureanhydride, wie

Tetrahydrophthalsäureanhydrid, Methyltetrahydrophthalsäureanhydrid, Hexahydrophthalsäureanhydrid, Methylhexahydrophthalsäureanhydrid, Endomethylentetrahydrophthalsäureanhydrid, Methyl-endomethylen-tetrahydrophthalsäureanhydrid (=Methylnadicanhydrid),

das Diels-Alder-Addukt aus 2 Mol Maleinsäureanhydrid und 1 Mol 1,4-Bis-(cyclopentadienyl)-2-buten, die eutektischen Gemische dieser Polycarbonsäureanhydride sowie Isomerengemische der methylsubstituierten Tetrahydrophthalsäureanhydride, oder gewisse aromatische Polycarbonsäureanhydride, wie Trimellitsäureanhydrid oder Pyromellitsäureanhydrid.

In den folgenden Beispielen stehen Teile für Gewichtsteile; Prozente bedeuten, falls nicht näher angegeben, Gewichtsprozente.

### Beispiel 1

N-(3'-Dimethylaminopropyl)-asparaginsäure-4-methylester

In einem Glaskolben, ausgerüstet mit Flügelrührer, Thermometer, Rückflußkühler und Tropftrichter, werden 286 g (2,0 Mol + 10% Überschuß) Maleinsäure-monomethylester vorgelegt und unter Eiswasserkühlung bei 6° – 11° C Innentemperatur 400 ml Triäthylamin innerhalb von 1 Stunde und 35 Minuten zugetropft. Anschließend wird die Kühlung entfernt, 204 g (2,0 Mol) 3-Dimethylaminopropylamin rasch zugegeben und das Gemisch 10 Minuten lang bei 87° – 90° C zur Reaktion gebracht. Man kühlt auf Raumtemperatur ab, vermischt das kristalline Rohprodukt mit 1 Liter Aceton, filtriert die Suspension und wäscht den Filterrückstand mit Aceton. Nach dem Trocknen bei 80° C im Vakuum werden 296 g (63,7% der Theorie) eines kristallinen Asparaginsäurederivats erhalten, das bei 171° – 172° C schmilzt.

Elementaranalyse:

| | | | |
|---|---|---|---|
| berechnet | 51,71% C | gefunden | 51,66% C |
| | 8,68% H | | 68,67% H |
| | 12,06% N | | 12,32% N |

Die 100-MHz-$^{1}$H-NMR- und Massenspektren stimmen mit folgender Struktur überein:

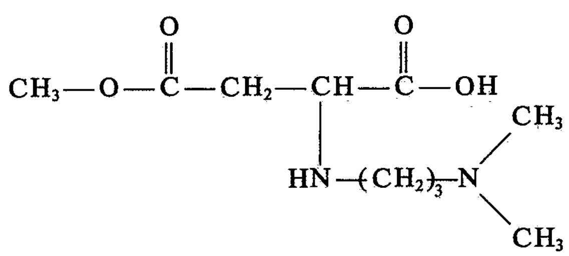

### Beispiel 2

N-(3'-Dimethylaminopropyl)-asparaginsäure-4-äthylester

Analog Beispiel 1 werden 360,3 g (2,5 Mol) Maleinsäure-monoäthylester vorgelegt und unter Kühlung 500 ml Triäthylamin zugetropft. Anschließend werden 306,5 g (2,5 Mol + 20% Überschuß) 3-Dimethylaminopropylamin zugegeben und das Reaktionsgemisch 50 Minuten lang bei 64° – 90° C umgesetzt. Das Rohprodukt wird gemäß Beispiel 1 aufgearbeitet, und man erhält 568,2 g (92,3% der Theorie) eines weißen, kristallinen Asparaginsäurederivats, dessen Schmelzpunkt 168° – 169° C beträgt.

Elementaranalyse:

| | | | |
|---|---|---|---|
| berechnet | 53,64% C | gefunden | 53,44% C |
| | 9,00% H | | 9,02% H |
| | 11,37% N | | 11,53% N |

Das 100-MHz-$^1$H-NMR-Spektrum deckt sich mit folgender Struktur:

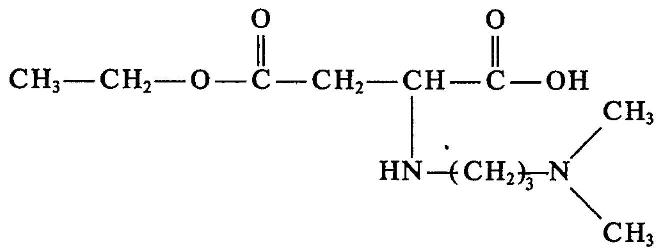

## Beispiel 3

### N-(3'-Dimethylaminopropyl)-asparaginsäure-4-n-butylester

In der in Beispiel 1 beschriebenen Apparatur werden 430,5 g (2,5 Mol) Maleinsäure-mono-n-butylester unter Kühlung innerhalb von 2 Stunden und 35 Minuten bei 4° – 10° C Innentemperatur mit 500 ml Triäthylamin versetzt. Anschließend gibt man rasch 306,5 g (2,5 Mol + 20% Überschuß) 3-Dimethylaminopropylamin zu und läßt das Gemisch 30 Minuten lang bei 90° – 95° C reagieren. Das Reaktionsprodukt wird analog Beispiel 1 aufgearbeitet, und es werden 595 g (86,8% der Theorie) eines kristallinen Asparaginsäurederivats erhalten, das bei 154° – 157,5° C schmilzt.

Elementaranalyse

| | | | | |
|---|---|---|---|---|
| berechnet | 56,65% C | | gefunden | 56,62% C |
| | 9,51% H | | | 9,65% H |
| | 10,16% N | | | 10,69% N |
| | 0,46% $H_2O$ | | | 0,46% $H_2O$ |

Das 100-MHz-$^1$H-NMR-Spektrum steht im Einklang mit folgender Struktur:

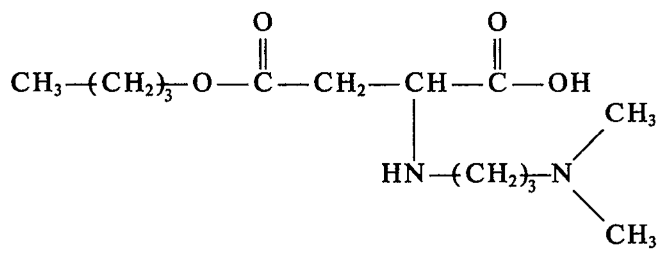

## Beispiel 4

### N-(3'-Dimethylaminopropyl)-asparaginsäure-4-isopropylester

395,2 g (2,5 Mol) Maleinsäure-mono-isopropylester, 500 ml Triäthylamin und 306,5 g (2,5 Mol + 20% Überschuß) 3-Dimethylaminopropylamin werden gemäß Beispiel 1 zur Reaktion gebracht und das Reaktionsprodukt anschließend analog gereinigt. Man erhält 616,4 g (94,7% der Theorie) eines weißen, kristallinen Asparaginsäurederivats, dessen Schmelzpunkt 163,5° – 165,5° C beträgt.

Elementaranalyse

| | | | | |
|---|---|---|---|---|
| berechnet | 55,12% C | | gefunden | 55,12% C |
| | 9,32% H | | | 9,13% H |
| | 10,71% N | | | 11,00% N |
| | 0,45% $H_2O$ | | | 0,45% $H_2O$ |

Das 100-MHz-¹H-NMR-Spektrum stimmt mit folgender Struktur überein:

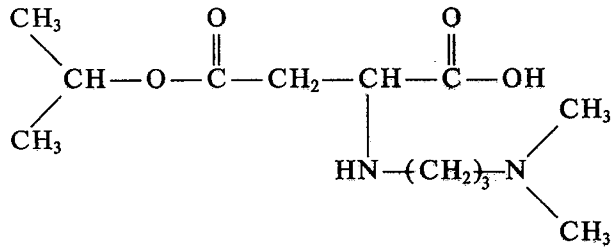

Beispiel 5

N-(3′-Dimethylaminopropyl)-asparaginsäure-4-cyclohexylester

Analog Beispiel 1 werden zu 99,1 g (0,5 Mol) Maleinsäure-mono-cyclohexylester unter Kühlung 100 ml Triäthylamin zugetropft und anschließend mit 61,3 g (0,5 Mol + 20% Überschuß) 3-Dimethylaminopropylamin 1 Stunde lang bei 80° – 93° C umgesetzt. Das Reaktionsprodukt wird wie in Beispiel 1 beschrieben gereinigt, und man erhält 109,9 g (73,2% der Theorie) des gewünschten Asparaginsäurederivats, dessen Schmelzpunkt 150° – 152,5° C beträgt.

Elementaranalyse

| | | | |
|---|---|---|---|
| berechnet | 59,98% C | gefunden | 60,10% C |
| | 9,40% H | | 9,50% H |
| | 9,33% N | | 9,62% N |

Das 100-MHz-¹H-NMR-Spektrum stimmt mit folgender Struktur überein:

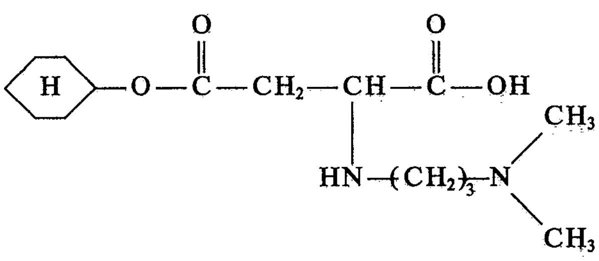

Beispiel 6

N-(3′-Methyläthylaminopropyl)-asparaginsäure-4-cyclohexylester

43,6 g (0,2 Mol + 10% Überschuß) Maleinsäure-monocyclohexylester, 40 ml Triäthylamin und 23,2 g (0,2 Mol) 3-(Methyläthylamino)-propylamin werden gemäß Beispiel 1 umgesetzt und analog aufgearbeitet. Man erhält 36,3 g (57,7% der Theorie) eines kristallinen Asparaginsäurederivats, das nach dem Umkristallisieren in Aceton bei 123,5° – 127,5° C schmilzt.

Elementaranalyse

| | | | |
|---|---|---|---|
| berechnet | 60,56% C | gefunden | 60,36% C |
| | 9,53% H | | 9,67% H |
| | 8,83% N | | 8,88% N |
| | 0,92% $H_2O$ | | 0,92% $H_2O$ |

Das 100-MHz-¹H-NMR-Spektrum deckt sich mit folgender Struktur:

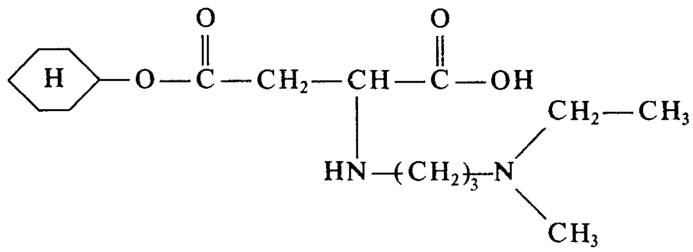

## Beispiel 7

### N-(3'-Diäthylaminopropyl)-asparaginsäure-4-methylester

71,5 g (0,5 Mol + 10% Überschuß) Maleinsäure-monomethylester werden, analog Beispiel 1, mit 100 ml Triäthylamin und 65,1 g (0,5 Mol) 3-Diäthylaminopropylamin umgesetzt. Das Reaktionsprodukt wird mit 500 ml Äther vermischt, filtriert und der Filterrückstand mit Äther gewaschen. Nach dem Trocknen bei 60° C im Vakuum wird das Rohprodukt in Aceton umkristallisiert, und man erhält 69,44 g (53,4% der Theorie) eines weißen, kristallinen Asparaginsäurederivats, dessen Schmelzpunkt 154,5° – 156,5° C beträgt.

Elementaranalyse

| | | | |
|---|---|---|---|
| berechnet | 55,37% C | gefunden | 55,46% C |
| | 9,29% H | | 9,21% H |
| | 10,76% N | | 10,75% N |

Das 100-MHz-$^1$H-NMR-Spektrum steht im Einklang mit folgender Struktur:

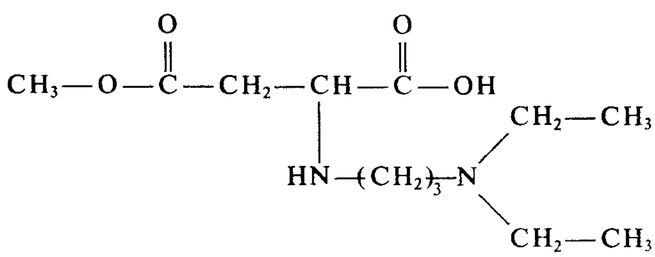

## Beispiel 8

### N-(3'-Piperidinopropyl)-asparaginsäure-4-methylester

Analog Beispiel 1 werden 28,6 g (0,2 Mol + 10% Überschuß) Maleinsäure-monomethylester, 40 ml Triäthylamin und 88,4 g (0,2 Mol) N-(3'-Aminopropyl)-piperidin umgesetzt und gereinigt. Die Ausbeute beträgt 36,7 g (67,4% der Theorie), und der Schmelzpunkt liegt bei 172° – 173° C.

Elementaranalyse

| | | | |
|---|---|---|---|
| berechnet | 57,06% C | gefunden | 57,25% C |
| | 8,91% H | | 9,09% H |
| | 10,24% N | | 10,45% N |
| | 0,48% $H_2O$ | | 0,48% $H_2O$ |

Das 100-MHz-$^1$H-NMR-Spektrum deckt sich mit folgender Struktur:

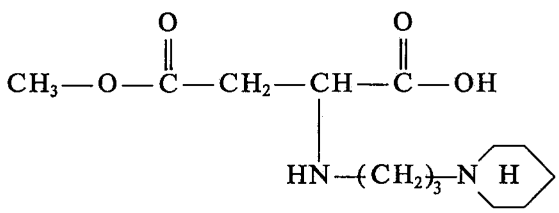

## Beispiel 9

N-3'-(2''-Methylimidazolyl)-propyl-asparaginsäure-4-benzylester

Ein Gemisch aus 82,4 g (0,4 Mol) Maleinsäure-monobenzylester und 30 ml Dioxan wird analog Beispiel 1 mit 80 ml Triäthylamin versetzt und anschließend mit 56,0 g (0,4 Mol) N-3'-Aminopropyl-2-methylimidazol während 20 Minuten bei 83°C zur Reaktion gebracht. Anschließend wird das Reaktionsgemisch auf Raumtemperatur abgekühlt, die flüssige Phase abdekantiert, das viskose Reaktionsgemisch mit 400 ml Diäthyläther versetzt und verrührt. Man dekantiert die ätherische Phase ab, löst den viskosen Rückstand bei 60°C in 270 ml Isopropanol und behandelt die Lösung mit Aktivkohle. Die Lösung wird filtriert, das Filtrat mit 270 ml Diäthyläther verdünnt und das Produkt bei 0°C zum Kristallisieren gebracht. Das auskristallisierte Produkt wird durch Filtration isoliert und bei 50°C im Vakuum getrocknet.

Es werden 56,3 g (40,7% der Theorie) des gewünschten Asparaginsäurederivats erhalten, das nach Umkristallisieren in Isopropylalkohol bei 134°C schmilzt.

Elementaranalyse

| | | | |
|---|---|---|---|
| berechnet | 62,59% C | gefunden | 62,25% C |
| | 6,71% H | | 6,59% H |
| | 12,17% N | | 12,34% N |

Die $^{13}C$- und 100-MHz-$^1H$-NMR-Spektren stehen im Einklang mit folgender Struktur:

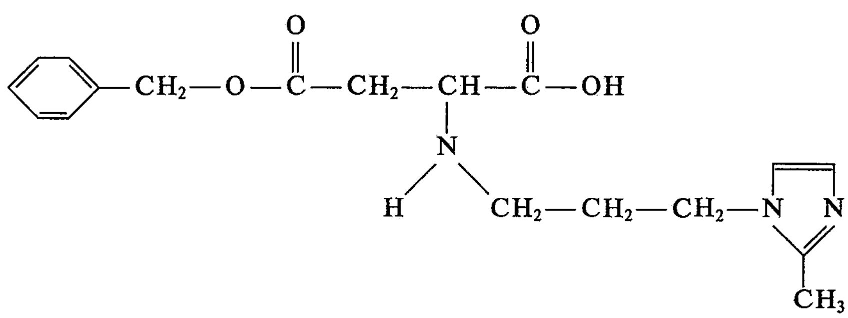

## Beispiel 10

N-(2'-Dimethylaminoäthyl)-asparaginsäure-4-methylester

28,6 g (0,22 Mol) Maleinsäure-monomethylester, 40 ml Triäthylamin und 17,6 g (0,20 Mol) 2-Dimethylaminoäthylamin werden analog Beispiel 1 zur Reaktion gebracht. Nach erfolgter Aufarbeitung gemäß Beispiel 1 erhält man 14,5 g (33,2% der Theorie) eines kristallinen Asparaginsäurederivats, das bei 164° – 166°C schmilzt.

Elementaranalyse

| | | | |
|---|---|---|---|
| berechnet | 49,01% C | gefunden | 49,05% C |
| | 8,37% H | | 8,14% H |
| | 12,70% N | | 12,69% N |
| | 1,06% $H_2O$ | | 1,06% $H_2O$ |

Die analytischen Daten stimmen mit folgender Struktur überein:

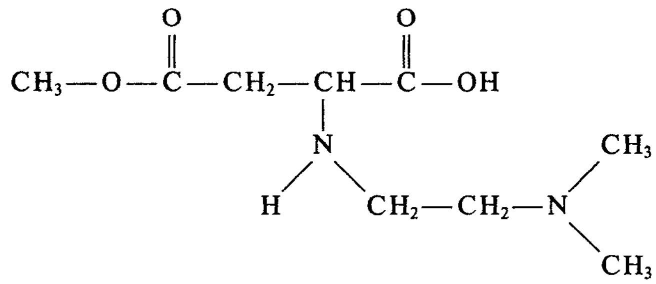

## Anwendungsbeispiele

### Beispiel I

Verwendung des N-(3'-Dimethylaminopropyl)-asparaginsäure-4-n-butyl-esters als latenter Beschleuniger in Epoxidharz-Dicyandiamid-Systemen

Zunächst wird eine Vergleichsformulierung hergestellt: Aus einem festen halogenierten Epoxidharz auf Basis von bromiertem Bisphenol A und Epichlorhydrin mit einem Epoxidgehalt von 1,92 Äquivalenten/kg und einem Bromgehalt von 21 Gew.-% wird eine 20%ige Lösung in Methyläthylketon hergestellt (Komponente I).

Das Härtungsmittel Dicyandiamid wird als 10%ige Lösung in Methylglykol eingesetzt (Komponente II).

Die Vergleichsformulierung besteht aus:

| | |
|---|---|
| Komponente I: | 62,5 g |
| Komponente II: | 15 g |
| Methylglykol: | 5 g |
| Benzyldimethylamin: | 0,1 g |

und stellt nach entsprechender Durchmischung eine homogene, klare Lösung dar (Formulierung A).

Es wird nun eine analoge Formulierung hergestellt, wobei anstelle des Benzyldimethylamins diesmal N-(3'-Dimethylaminopropyl)-asparaginsäure-4-n-butylester als Beschleuniger verwendet wird:

| | |
|---|---|
| Komponente I: | 62,5 g |
| Komponente II: | 15 g |
| Methylglykol: | 5 g |
| N-(3'-Dimethylaminopropyl)-asparaginsäure-4-n-butylester | 0,25 g |

Der feste Asparaginsäureester wird in 5 g Methylglykol gelöst. Nach entsprechender Durchmischung liegt auch hier eine homogene, klare Lösung vor (Formulierung B).

### Vergleichsmessungen

Gemessen wird die Gelierzeit bei 100° und 150° C (auf der Gelierzeitplatte) sowie die Gebrauchsdauer bei 40° C im Höppler Viskositätsröhrchen. Als Endpunkt der Gebrauchsdauer wird die Zeit angegeben, in der eine Verdoppelung der Ausgangsviskosität stattfand.

| | Formulierung | | |
|---|---|---|---|
| | A | B | Δ % |
| Gelierzeit 100°C (Sekunden) | 9 000 | 11 400 | |
| Gelierzeit 150°C (Sekunden) | 660 | 560 | |
| Latenzfaktor (100°/150°C)**) | 13,6 | 20,4 | +50 |
| Gebrauchsdauer bei 40°C (Stunden) | 268 | 340 | +26,9 |

**) Verhältnis der Gelierzeiten 100°/150°C.

Aus dem Vergleich geht hervor, daß man bei der Verwendung des erfindungsgemäßen N-(3'-Dimethylaminopropyl)-asparaginsäureesters als Härtungsbeschleuniger in Epoxidharz-Dicyandiamid-Mischungen eine Verkürzung der Gelierzeit bei 150° C und eine Verlängerung der Gelierzeit bei 100° C, was von der Applikation her erwünscht ist, erzielt. Außerdem zeigt die den erfindungsgemäßen Härtungsbeschleuniger enthaltende Harz-Härter-Mischung eine vorteilhafte Verlängerung der Gebrauchsdauer bei 40° C.

Beispiel II

Verwendung des N-(3'-Dimethylaminopropyl)-asparaginsäure-4-methylesters als latenter Beschleuniger für ein Pulversystem auf Basis von Epoxidharz und Carbonsäureanhydrid

Dieses besteht aus einem festen Epoxidharz auf Basis von Bisphenol A und Epichlorhydrin mit einem Epoxidgehalt von 1,1 Äquivalenten/kg, Trimellitsäureanhydrid als Härtungsmittel und Imidazol:

| | |
|---|---|
| Epoxidharz: | 50 g |
| Trimellitsäureanhydrid: | 5,5 g |
| Imidazol | 0,55 g |

Zur Herstellung des Pulvers wird das Epoxidharz während 16 Stunden in der Kugelmühle vorgemahlen. Anschließend werden die anderen beiden festen Komponenten beigegeben und das Ganze dann während 5 Stunden gemahlen und gleichzeitig gründlich durchgemischt.

Formulierung B
(erfindungsgemäß)

Diese wird in ganz analoger Weise hergestellt wie die obige Formulierung A und besteht aus:

| | |
|---|---|
| Epoxidharz: | 50 g |
| Trimellitsäureanhydrid: | 5,5 g |
| N-(3-Dimethylaminopropyl)-asparaginsäure-4-methylester | 1,375 g |

Vergleichsmessungen

Gemessen wird die Gelierzeit bei 120° und 180° C (Gelierzeitplatte) sowie die Lagerstabilität bei 40° C. Letztere wird beurteilt, indem die Gelierzeit bei 180° C nach einer Lagerzeit von 30 Tagen nochmals gemessen wird. In der Praxis darf es bei einem Pulversystem zu keiner wesentlichen Reaktivitätsänderung nach Ablauf dieser Zeit kommen. Das Pulversystem muß auch einwandfrei rieselfähig bleiben.

| | Formulierung | | |
|---|---|---|---|
| | A | B | Δ % |
| Gelierzeit 120°C (Sekunden) | 315 | 1485 | |
| Gelierzeit 180°C (Sekunden) | 70 | 70 | |
| Latenzfaktor (120°/180°C)*) | 4,5 | 21,2 | +371% |
| Gelierzeit bei 180°C nach 30 Tagen Lagerung bei 40°C | 40 | 70 | |
| Rieselfähigkeit nach 30 Tagen Lagerung bei 40°C | gut | gut | |

*) Verhältnis der Gelierzeiten 120°/180°C.

Während beide Pulversysteme bei 180° C gleiche Gelierzeiten aufweisen, weist die Formulierung B, die einen erfindungsgemäßen Asparaginsäureester anstelle des Imidazols als Beschleuniger enthält, bei 120° C eine fast fünffach längere Gelierzeit auf. Dies bedeutet, daß mit der Formulierung B ein wesentlich höherer Latenzfaktor erzielt wird, was bei einer Applikation, beispielsweise als Pulverlack, erwünscht ist, da damit eine größere Sicherheit beim Extrudieren erreicht wird.

Ferner weist die Formulierung B eine unvergleichbar bessere Lagerstabilität bei 40°C als die Vergleichsformulierung A auf.

## Patentansprüche

1. N-substituierte Asparaginsäuremonoester der Formel I

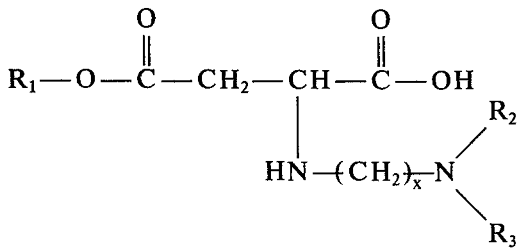

(I)

worin

$R_1$ ein gegebenenfalls Äthersauerstoffatome enthaltendes Alkyl mit 1 bis 12 C-Atomen, Cyclohexyl, Phenyl, Tolyl oder Benzyl bedeutet,
x für die Zahl 2 oder 3 steht,
$R_2$ und $R_3$ unabhängig voneinander je ein Methyl oder Äthyl oder zusammen mit dem N-Atom einen N-heterocyclischen Ring der Formel

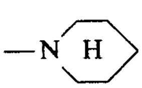

oder

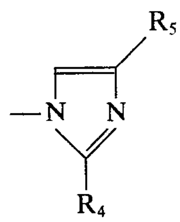

bedeuten, worin $R_4$ und $R_5$ unabhängig voneinander je ein Wasserstoffatom, Methyl oder Äthyl bedeuten.

2. Asparaginsäuremonoester gemäß Anspruch 1, worin in der Formel I $R_1$ ein Alkyl mit 1 bis 6 C-Atomen oder Cyclohexyl bedeutet, x für die Zahl 3 steht und $R_2$ und $R_3$ je ein Methyl oder Äthyl oder zusammen mit dem N-Atom einen N-heterocyclischen Ring der Formel

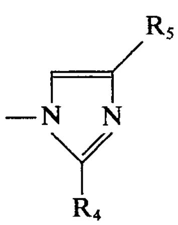

bedeuten, worin $R_4$ für Methyl steht und $R_5$ ein Wasserstoffatom oder Äthyl bedeutet.

3. N-(3'-Dimethylaminopropyl)-asparaginsäure-4-methylester als Verbindung der Formel I gemäß Anspruch 1.

4. N-(3'-Dimethylaminopropyl)-asparaginsäure-4-n-butylester als Verbindung der Formel I gemäß Anspruch 1.

5. N-(3'-Dimethylaminopropyl)-asparaginsäure-4-isopropylester als Verbindung der Formel I gemäß Anspruch 1.

6. N-(3'-Methyläthylaminopropyl)-asparaginsäure-4-cyclohexylester als Verbindung der Formel I gemäß Anspruch 1.

7. N-(3'-Diäthylaminopropyl)-asparaginsäure-4-methylester als Verbindung der Formel I gemäß Anspruch 1.

8. N-(3'-Piperidinopropyl)-asparaginsäure-4-methylester als Verbindung der Formel I gemäß

Anspruch 1.

9. Verfahren zur Herstellung von Asparaginsäuremonoester der Formel I gemäß Anspruch 1, dadurch gekennzeichnet, daß man an 1 Mol eines Maleinsäuremonoesters der Formel II

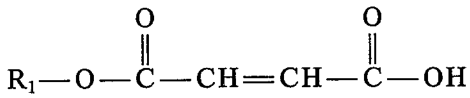

(II)

worin $R_1$ die gleiche Bedeutung wie in Formel I hat, 1 Mol eines Amins der Formel III

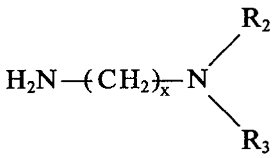

(III)

worin $R_2$, $R_3$ und x die gleiche Bedeutung wie in Formel I haben, in Gegenwart eines tertiären Amins anlagert.

10. Verwendung der Asparaginsäuremonoester der Formel I gemäß Anspruch 1 als Beschleuniger in härtbaren Epoxidharzmischungen, die Dicyandiamid oder ein Polycarbonsäureanhydrid als Härtungsmittel für Epoxidharze enthalten.

**Claims**

1. A N-substituted aspartic acid monoester of the formula I

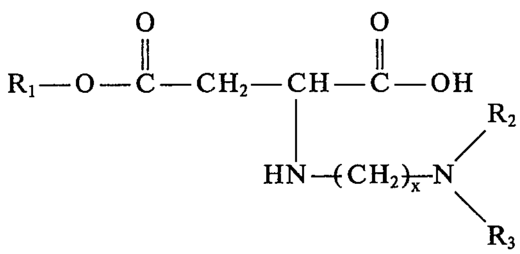

(I)

wherein

$R_1$ is an alkyl group which has 1 to 12 C atoms and which optionally contains ether oxygen atoms, or it is cyclohexyl, phenyl, tolyl or benzyl,
x is the number 2 or 3,
$R_2$ and $R_3$ independently of one another are each methyl or ethyl, or together with the N atom they form an N-heterocyclic ring of the formula

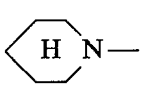

or

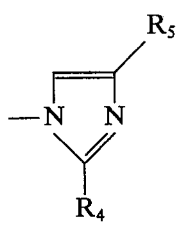

wherein $R_4$ and $R_5$ independently of one another are each a hydrogen atom, methyl or ethyl.

2. An aspartic acid monoester according to Claim 1, wherein, in the formula I, $R_1$ is an alkyl group having 1 to 6 C atoms or cyclohexyl, x is the number 3, and $R_2$ and $R_3$ are each a methyl or ethyl group, or together with the N atom they form an N-heterocyclic ring of the formula

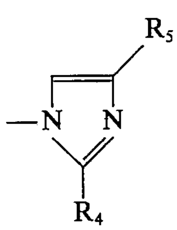

wherein $R_4$ is methyl, and $R_5$ is a hydrogen atom or ethyl.

3. N-(3'-Dimethylaminopropyl)-aspartic acid-4-methyl ester as compound of the formula I according to Claim 1.

4. N-(3'-Dimethylaminopropyl)-aspartic acid 4-n-butyl ester as compound of the formula I according to Claim 1.

5. N-(3'-Dimethylaminopropyl)-aspartic acid-4-isopropyl ester as compound of the formula I according to Claim 1.

6. N-(3'-Methylethylaminopropyl)-aspartic acid-4-cyclohexyl ester as compound of the formula I according to Claim 1.

7. N-(3'-Diethylaminopropyl)-aspartic acid-4-methyl ester as compound of the formula I according to Claim 1.

8. N-(3'-Piperidinopropyl)-aspartic acid-4-methyl ester as compound ot the formula I according to Claim 1.

9. A process for producing an aspartic acid monoester of the formula I according to Claim 1, which process comprises adding, by means of an addition reaction, to 1 mol of a maleic acid monoester of the formula II

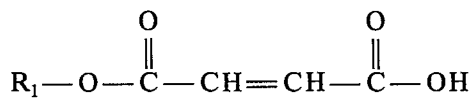

(II)

wherein $R_1$ has the same meaning as in the formula I, 1 mol of an amine of the formula III

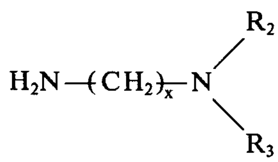

(III)

wherein $R_2$, $R_3$ and x have the same meanings as in the formula I, in the presence of a tertiary amine.

10. Use of an aspartic acid monoester of the formula I according to Claim 1 as a curing accelerator in curable epoxide resin mixtures containing dicyandiamide or a polycarboxylic acid anhydride as curing agent for epoxide resins.

## Revendications

1. Monoesters d'acides aspartiques substitués à l'azote, qui répondent à la formule (I):

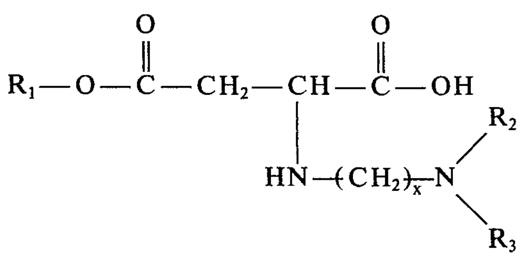

(I)

dans laquelle:

$R_1$ représente un radical alkyle en $C_1-C_{12}$ qui renferme éventuellement des atomes d'oxygène de fonction éther, ou un radical cyclohexyle, phényle, tolyle ou benzyle,

x représente un nombre égal à 2 ou à 3, et

$R_2$ et $R_3$ représentent chacun, indépendamment l'un de l'autre, un radical méthyle ou éthyle ou forment ensemble et avec l'atome d'azote un noyau hétérocyclique azoté répondant à l'une des formules:

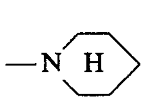

et

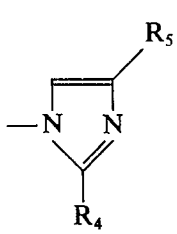

où $R_4$ et $R_5$ représentent chacun, indépendamment l'un de l'autre, un atome d'hydrogène, un radical méthyle ou un radical éthyle.

2. Monoesters aspartiques selon la revendication 1, dans la formule (I) desquels $R_1$ représente un alkyle contenant de 1 à 6 atomes de carbone ou un cyclohexyle, x est égal à 3 et $R_2$ et $R_3$ représentent chacun un méthyle ou un éthyle ou forment ensemble et avec l'atome d'azote un noyau hétérocyclique azoté répondant à la formule:

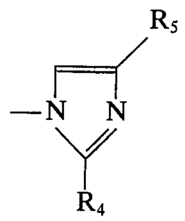

dans laquelle $R_4$ représente un méthyle et $R_5$ représente un atome d'hydrogène ou un éthyle.

3. Composé de formule (I) selon la revendication 1, en l'espèce le N-(diméthylamino-3 propyl)-aspartate-4 de méthyle.

4. Composé de formule (I) selon la revendication 1, en l'espèce le N-(diméthylamino-3 propyl)-aspartate-4 de n-butyle.

5. Composé de formule (I) selon la revendication 1, en l'espèce le N-(diméthylamino-3 propyl)-aspartate-4 d'isopropyle.

6. Composé de formule (I) selon la revendication 1, en l'espèce le N-(méthyléthylamino-3 propyl)-aspartate-4 de cyclohexyle.

7. Composé de formule (I) selon la revendication 1, en l'espèce le N-(diéthylamino-3 propyl)-aspartate-4 de méthyle.

8. Composé de formule (I) selon la revendication 1, en l'espèce le N-(pipéridino-3 propyl)-aspartate-4 de méthyle.

9. Procédé de préparation de monoesters aspartiques de formule (I) selon la revendication 1, procédé caractérisé en ce qu'on fixe sur 1 mole d'un monoester maléique répondant à la formule (II):

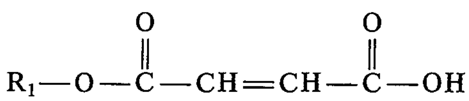

(II)

dans laquelle $R_1$ a la même signification que dans la formule (I), 1 mole d'une amine répondant à la formule (III):

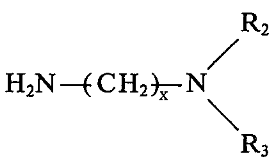

(III)

dans laquelle $R_2$, $R_3$ et x ont les mêmes significations que dans la formule (I), en présence d'une amine tertiaire.

10. Application des monoesters aspartiques de formule (I) selon la revendication 1 comme accélérateurs dans des mélanges durcissables à base de résines époxydiques qui contiennent de la cyanoguanidine ou un anhydride d'acide polycarboxylique comme durcisseur pour résines époxydiques.